(19) Europäisches Patentamt
European Patent Office
Office européen des brevets

(11) **EP 4 241 731 A1**

(12) **EUROPEAN PATENT APPLICATION**
published in accordance with Art. 153(4) EPC

(43) Date of publication:
**13.09.2023 Bulletin 2023/37**

(21) Application number: **20965217.1**

(22) Date of filing: **16.12.2020**

(51) International Patent Classification (IPC):
**A61C 9/00** (2006.01)  **G16H 30/40** (2018.01)
**G16H 30/00** (2018.01)  **A61B 5/00** (2006.01)
**G06T 7/00** (2017.01)  **G06N 3/08** (2023.01)
**A61B 18/20** (2006.01)

(52) Cooperative Patent Classification (CPC):
**A61B 5/00; A61B 18/20; A61C 9/00; G06N 3/08;**
**G06T 7/00; G16H 30/00; G16H 30/40**

(86) International application number:
**PCT/KR2020/018432**

(87) International publication number:
**WO 2022/124462 (16.06.2022 Gazette 2022/24)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **10.12.2020 KR 20200172656**

(71) Applicant: **Imagoworks Inc.**
**Seoul 02455 (KR)**

(72) Inventors:
• KIM, Youngjun
  Seoul 06610 (KR)
• SHIN, Bonjour
  Seoul 02740 (KR)
• KIM, Hannah
  Seoul 05400 (KR)
• CHOI, Jinhyeok
  Seoul 06295 (KR)

(74) Representative: **BCKIP Part mbB**
**Siegfriedstraße 8**
**80803 München (DE)**

(54) **METHOD FOR AUTOMATICALLY DETECTING LANDMARK IN THREE-DIMENSIONAL DENTAL SCAN DATA, AND COMPUTER-READABLE RECORDING MEDIUM WITH PROGRAM FOR EXECUTING SAME IN COMPUTER RECORDED THEREON**

(57) A method for automatically detecting a landmark in three-dimensional (3D) dental scan data includes projecting 3D scan data to generate a two-dimensional (2D) depth image, determining full arch data obtained by scanning all teeth of a patient and partial arch data obtained by scanning only a part of teeth of the patient by applying the 2D depth image to a convolutional neural network model, detecting a 2D landmark in the 2D depth image using a fully-connected convolutional neural network model and back-projecting the 2D landmark onto the 3D scan data to detect a 3D landmark of the 3D scan data.

FIG. 1

START

PROJECTING 3D SCAN DATA TO GENERATE 2D DEPTH IMAGE — S100

DETERMINING FULL ARCH DATA AND PARTIAL ARCH DATA BY APPLYING 2D DEPTH IMAGE TO CONVOLUTIONAL NEURAL NETWORK — S200

DETECTING 2D LANDMARK BY APPLYING 2D DEPTH IMAGE TO FULLY-CONNECTED CONVOLUTIONAL NEURAL NETWORK — S300

BACK-PROJECTING 2D LANDMARK ONTO 3D SCAN DATA TO DETECT 3D LANDMARK OF 3D SCAN DATA — S400

END

**Description**

[TECHNICAL FIELD]

The present inventive concept relates to a method for automatically detecting a landmark in three-dimensional dental scan data and a non-transitory computer-readable storage medium having stored thereon program instructions of the method for automatically detecting a landmark in three-dimensional dental scan data. More particularly, the present inventive concept relates to a method for automatically detecting a landmark in three-dimensional dental scan data reducing time and effort for registration of a dental CT image and a digital impression model and a non-transitory computer-readable storage medium having stored thereon program instructions of the method for automatically detecting a landmark in three-dimensional dental scan data.

[BACKGROUND]

CT (Computed Tomography) or CBCT (Cone Beam Computed Tomography) (hereafter collectively referred to as CT) data, which are three-dimensional (3D) volume data required when diagnosing oral and maxillofacial conditions or establishing surgery and treatment plans in dentistry, plastic surgery, etc., includes not only hard tissue such as a bone or tooth, but also various information such as a soft tissue such as a tongue or lip, and a position and shape of a neural tube existing inside a bone. However, due to metallic substances present in the oral cavity such as implants, orthodontic devices, dental crowns, etc. which the patient has previously undergone treatment, metal artifact may occur in CT, which is an X-ray based image, so that teeth and an area adjacent to the teeth may be greatly distorted. Thus, the identification and diagnosis of teeth may be difficult. In addition, it is difficult to specify a shape of a gum or a boundary between the gum and the teeth. A 3D digital scan model may be acquired and used to compensate for such limited tooth and oral cavity information. The 3D digital scan model may be obtained by directly scanning the oral cavity of the patient or by scanning a plaster impression model of the patient. The 3D digital scan model may be data having a 3D model file format (hereafter referred to as scan data) such as stl, obj and ply and including point and plane information.

For using the scan data along with the CT data, a registration process of overlapping the data of different modalities may be performed. Generally, a user may manually set landmarks on the scan data and the CT data for the same locations, and then the scan data may be matched on the CT data based on the landmarks. In addition, scan data of a same patient acquired at different times may be matched in the same way to confirm treatment progress or to compare before and after treatment. The registration result is an important basic data for treatment, surgery, etc., so that increasing the accuracy of registration is very important. In particular, in a case of an implant, the registration result is a basis for a plan to place an implant in an optimal position by identifying the location of neural tubes, tissues, etc., so that the position of the landmark which is a registration basis requires high accuracy. However, manually marking landmarks on a consistent basis or at corresponding locations in two different types of 3D data is difficult, takes a lot of time, and varies among users.

If markers are directly attached to the oral cavity of the patient to generate the scan data to obtain the landmark, it may cause discomfort to the patient, and since the inside of the oral cavity is a soft tissue, it may be difficult to fix the marker.

[DETAILED EXPLANATION OF THE INVENTION]

[TECHNICAL PURPOSE]

The purpose of the present inventive concept is providing a method for automatically detecting a landmark in three-dimensional (3D) dental scan data capable of automatically detecting a landmark in 3D scan data to reduce time and effort for registration of a dental CT image and the 3D scan data.

Another purpose of the present inventive concept is providing a non-transitory computer-readable storage medium having stored thereon program instructions of the method for automatically detecting the landmark in the 3D dental scan data.

[TECHNICAL SOLUTION]

In an example method for automatically detecting a landmark in three-dimensional (3D) dental scan data according to the present inventive concept, the method includes projecting 3D scan data to generate a two-dimensional (2D) depth image, determining full arch data obtained by scanning all teeth of a patient and partial arch data obtained by scanning only a part of teeth of the patient by applying the 2D depth image to a convolutional neural network model, detecting a 2D landmark in the 2D depth image using a fully-connected convolutional neural network model and back-projecting the 2D landmark onto the 3D scan data to detect a 3D landmark of the 3D scan data.

In an embodiment, the projecting the 3D scan data may include determining a projection direction vector by a principal component analysis.

In an embodiment, the determining the projection direction vector may include moving($X' = X - \bar{X}$) a

$$X = \begin{bmatrix} x_1 x_2 \cdots x_n \\ y_1 y_2 \cdots y_n \\ z_1 z_2 \cdots z_n \end{bmatrix}$$

matrix                     of a set $\{i \in \{1, 2, \cdots, n\} \mid p_i(x_i, y_i, z_i)\}$ of coordinates of n 3D points of the 3D scan data based on an average value $\bar{X}$ of

$$X = \begin{bmatrix} x_1 & x_2 & \cdots & x_n \\ y_1 & y_2 & \cdots & y_n \\ z_1 & z_2 & \cdots & z_n \end{bmatrix}$$

, calculating a covariance

$$\Sigma = cov(X') = \frac{1}{n-1} X' X'^T$$ for the coordinates of the n 3D points, operating ($\Sigma A = A\Lambda$) eigen decomposition of $\Sigma$ and determining the projection direction vector based on a direction vector $w_3$ having the smallest eigenvalue $\lambda$ among $w_1 = \{w_{1p}, w_{1q}, w_{1r}\}, w_2 = \{w_{2p}, w_{2q}, w_{2r}\}, w_3 = \{w_{3p}, w_{3q}, w_{3r}\}$.

$$A = \begin{bmatrix} w_{1p} & w_{2p} & w_{3p} \\ w_{1q} & w_{2q} & w_{3q} \\ w_{1r} & w_{2r} & w_{3r} \end{bmatrix}$$

Herein, and

$$\Lambda = \begin{bmatrix} \lambda_1 & 0 & 0 \\ 0 & \lambda_2 & 0 \\ 0 & 0 & \lambda_3 \end{bmatrix}$$ .

In an embodiment, the determining the projection direction vector may include determining $w_3$ as the projection direction vector when $\overline{\eta}$ is an average of normal vectors of the 3D scan data and $w_3 \cdot \overline{\eta} > 0$, and determining $-w_3$ as the projection direction vector when $\overline{\eta}$ is the average of the normal vectors of the 3D scan data and $w_3 \cdot \overline{\eta} \leq 0$.

In an embodiment, the 2D depth image may be generated on a projection plane, and the projection plane is defined at a location separated by a predetermined distance from the 3D scan data with the projection direction vector as a normal vector.

In an embodiment, the 2D landmark may be back-projected in a direction opposite to the projection direction vector onto the 3D scan data to detect the 3D landmark.

In an embodiment, e convolutional neural network model may include a feature extractor configured to extract a feature of the 2D depth image and a classifier configured to calculate a score for arch classification information based on the feature extracted by the feature extractor.

In an embodiment, the feature extractor may include a convolution layer including a process of extracting features of the 2D depth image and a pooling layer including a process of culling the extracted features into categories.

In an embodiment, the detecting the 2D landmark may include detecting the 2D landmark using a first fully-connected convolutional neural network model trained using full arch training data when the 2D depth image is the full arch data and detecting the 2D landmark using a second fully-connected convolutional neural network model trained using partial arch training data when the 2D depth image is the partial arch data.

In an embodiment, each of the first fully-connected convolutional neural network model and the second fully-connected convolutional neural network model may operate a convolution process extracting a landmark feature from the 2D depth image; and a deconvolution process adding landmark location information to the landmark feature.

In an embodiment, the convolution process and the deconvolution process may be repeatedly operated in the first fully-connected convolution neural network model. The convolution process and the deconvolution process may be repeatedly operated in the second fully-connected convolution neural network model. A number of the repeated operation of the convolution process and the deconvolution process in the first fully-connected convolution neural network model may be different from a number of the repeated operation of the convolution process and the deconvolution process in the second fully-connected convolution neural network model.

In an embodiment, the number of the repeated operation of the convolution process and the deconvolution process in the first fully-connected convolution neural network model may be greater than the number of the repeated operation of the convolution process and the deconvolution process in the second fully-connected convolution neural network model.

In an embodiment, the detecting the 2D landmark may further include training the convolutional neural network. The training the convolutional neural network may include receiving a training 2D depth image and user-defined landmark information. The user-defined landmark information may include a type of a training landmark and correct location coordinates of the training landmark in the training 2D depth image.

In an embodiment, the fully-connected convolutional neural network model may operate a convolution process extracting a landmark feature from the 2D depth image and a deconvolution process adding landmark location information to the landmark feature.

In an embodiment, a result of the deconvolution process may be a heat map corresponding to the number of the 2D landmarks.

In an embodiment, pixel coordinate having a largest value in the heat map may represent a location of the 2D landmark.

In an embodiment, a program for executing the method for automatically detecting the landmark in the 3D dental scan data on a computer may be recorded on a computer-readable recording medium.

[EFFECT OF THE INVENTION]

According to the method for automatically detecting the landmark in three-dimensional (3D) dental scan data, the landmark in the 3D scan data is automatically detected using a deep learning so that effort and time for generating the landmark in the 3D scan data may be reduced and an accuracy of the landmark in the 3D scan data may be enhanced.

In addition, the landmark in the 3D scan data is automatically detected using a deep learning so that an accuracy of the registration of the dental CT image and the 3D scan data may be enhanced and time and effort for the registration of the dental CT image and the 3D scan data may be reduced.

[BRIEF EXPLANATION OF THE DRAWINGS]

FIG. 1 is a flowchart diagram illustrating a method for automatically detecting a landmark in three-dimensional (3D) dental scan data according to an embodiment of the present inventive concept.
FIG. 2 is a perspective view illustrating an example of a landmark of the 3D scan data.
FIG. 3 is a conceptual diagram illustrating a method of generating a two-dimensional (2D) depth image by projecting the 3D scan data.
FIG. 4 is a perspective view illustrating a projection direction when generating the 2D depth image.
FIG. 5 is a perspective view illustrating a projection direction when generating the 2D depth image.
FIG. 6 is a plan view illustrating an example of the 2D depth image.
FIG. 7 is a plan view illustrating an example of the 2D depth image.
FIG. 8 is a perspective view illustrating full arch data and partial arch data.
FIG. 9 is a conceptual diagram illustrating a convolutional neural network distinguishing the full arch data and the partial arch data.
FIG. 10 is a conceptual diagram illustrating an example of training data of the convolutional neural network detecting a 2D landmark.
FIG. 11 is a conceptual diagram illustrating the convolutional neural network detecting the 2D landmark.
FIG. 12 is a conceptual diagram illustrating a first landmark detector for the full arch data and a second landmark detector for the partial arch data.
FIG. 13 is a plan view illustrating an example of the 2D landmark.
FIG. 14 is a conceptual diagram illustrating a method of generating the 3D landmark by back-projecting the 2D landmark onto the 3D scan data.

[BEST MODE FOR CARRYING OUT THE INVENTION]

The present inventive concept now will be described more fully hereinafter with reference to the accompanying drawings, in which exemplary embodiments of the present invention are shown. The present inventive concept may, however, be embodied in many different forms and should not be construed as limited to the exemplary embodiments set forth herein.

Rather, these exemplary embodiments are provided so that this disclosure will be thorough and complete, and will fully convey the scope of the present invention to those skilled in the art.

It will be understood that, although the terms first, second, third, etc. may be used herein to describe various elements, components, regions, layers and/or sections, these elements, components, regions, layers and/or sections should not be limited by these terms. These terms are only used to distinguish one element, component, region, layer or section from another region, layer or section. Thus, a first element, component, region, layer or section discussed below could be termed a second element, component, region, layer or section without departing from the teachings of the present invention.

It will be understood that when an element or layer is referred to as being "connected to" or "coupled to" another element or layer, it can be directly connected or coupled to the other element or layer or intervening elements or layers may be present. In contrast, when it is referred that an element is "directly connected to" or "directly coupled to" another element or layer, there are no intervening elements or layers present. Other expressions describing the relationship between elements, such as "between" and "directly between" or "adjacent to" and "directly adjacent to", etc., should be interpreted similarly. Like numerals refer to like elements throughout. As used herein, the term "and/or" includes any and all combinations of one or more of the associated listed items.

The terminology used herein is for the purpose of describing particular exemplary embodiments only and is not intended to be limiting of the present invention. As used herein, the singular forms "a," "an" and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof.

Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

All methods described herein can be performed in a suitable order unless otherwise indicated herein or otherwise clearly contradicted by context. The use of any and all examples, or exemplary language (e.g., "such as"), is intended merely to better illustrate the invention and does not pose a limitation on the scope of the invention unless otherwise claimed. No language in the specification should be construed as indicating any non-claimed element as essential to the practice of the inventive concept as used herein.

Hereinafter, preferred embodiments of the present inventive concept will be explained in detail with reference to the accompanying drawings. The same reference numerals are used for the same elements in the drawings, and duplicate explanations for the same elements may be omitted.

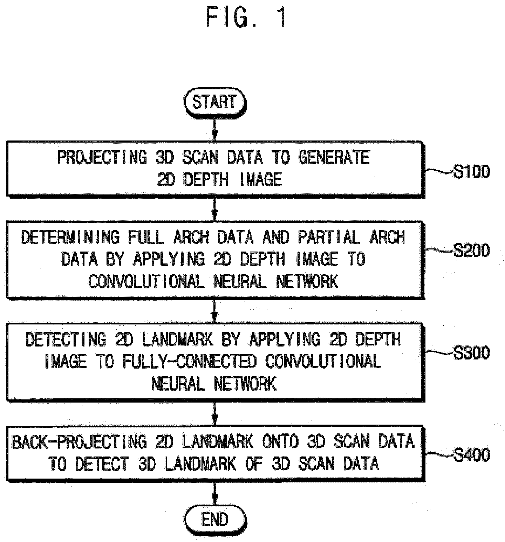

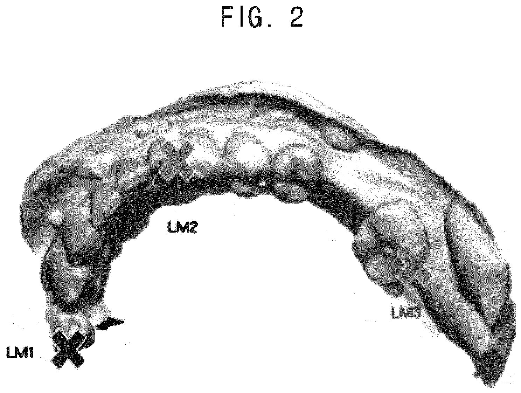

FIG. 1 is a flowchart diagram illustrating a method for automatically detecting a landmark in three-dimensional (3D) dental scan data according to an embodiment of the present inventive concept. FIG. 2 is a perspective view illustrating an example of a landmark of the 3D scan data.

Referring to FIGS. 1 and 2, the method for automatically detecting the landmark in the 3D dental scan data may include projecting the 3D scan data to generate a two-dimensional (2D) depth image (operation S 100), determining full arch data and partial arch data by applying the 2D depth image to a convolutional neural network (operation S200), detecting a 2D landmark by applying the 2D depth image to a fully-connected convolutional neural network (operation S300) and back-projecting the 2D landmark onto the 3D scan data to detect a 3D landmark of the 3D scan data (operation S400).

The generating the two-dimensional (2D) depth image (operation S100) may be an operation of imaging the depth of 3D scan data for a virtual camera. In the 3D scan data classification operation (operation S200), the 3D scan data may be classified into the full arch data and the partial arch data according to a shape of a scanned region. The 2D landmark automatic detection operation (operation S300) may be an operation of detecting the landmark from the 2D image using a fully-connected convolutional neural network deep learning model. In the landmark 3D projection operation (operation S400), the 2D landmark detected in the 2D landmark automatic detection operation (operation S300) may be converted into 3D and reflected in the 3D scan data.

FIG. 2 illustrates three landmarks LM1, LM2 and LM3 of the 3D scan data. In the present embodiment, the landmarks may be disposed at regular intervals or on a surface of preset teeth (incisors, canines, molars, etc.) so that a shape of a dental arch may be estimated. The landmarks may be automatically and simultaneously detected by applying the same method to all landmarks without additional processing according to locations or characteristics of the landmarks.

The landmarks of the 3D scan data may be points representing specific positions of teeth. For example, the landmarks of the 3D scan data may include three points LM1, LM2 and LM3. Herein, the 3D scan data may represent patient's maxilla data or the patient's mandible data. For example, the first landmark LM1 and the third landmark LM3 of the 3D scan data may represent the outermost points of the teeth of the 3D scan data in the lateral direction, respectively. The second landmark LM2 of the 3D scan data may be a point between the first landmark LM1 and the third landmark LM3 in an arch including the first landmark LM1 and the third landmark LM3. For example, the second landmark LM2 of the 3D scan data may correspond to a point between two central incisors of the patient.

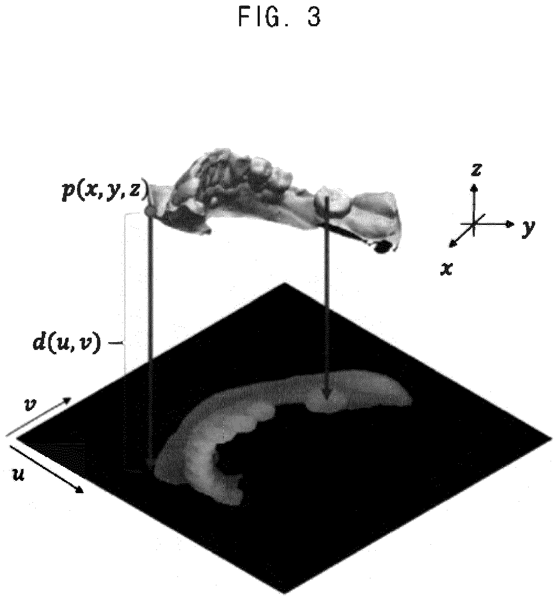

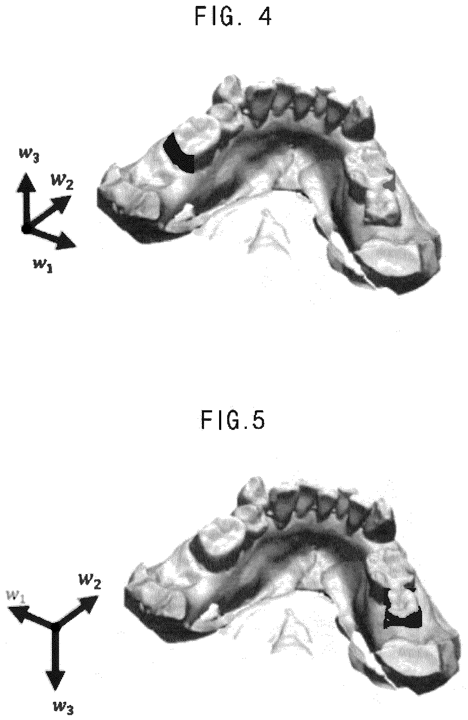

FIG. 3 is a conceptual diagram illustrating a method of generating a two-dimensional (2D) depth image by projecting the 3D scan data. FIG. 4 is a perspective view illustrating a projection direction when generating the 2D depth image. FIG. 5 is a perspective view illustrating a projection direction when generating the 2D depth image.

Referring to FIGS. 1 to 5, the depth image is an image representing vertical distance information between each 3D point $p(x,y,z)$ of the scan data and a plane UV defined by a principal component analysis of the scan data when the 3D scan data is projected on to a 2D plane. A pixel value of a 2D image represents a distance $d(u,v)$ from the 2D plane defined above to the surface of the scan data.

Herein, the principal component analysis (PCA) may be performed to determine the projection direction and a projection plane. First, the data X are moved based

$$X = \begin{bmatrix} x_1 x_2 \cdots x_n \\ y_1 y_2 \cdots y_n \\ z_1 z_2 \cdots z_n \end{bmatrix}$$

on an average value $\overline{X}$ of a matrix of a set $\{i \in \{1,2, \cdots, n\} \mid p_i(x_i, y_i, z_i)\}$ of coordinates of n 3D points of the scan data $(X' = X - \overline{X})$.

Then, a covariance

$$\Sigma = cov(X') = \frac{1}{n-1} X' X^T$$

for the coordinates of the n 3D points is obtained. The covariance may represent how the coordinates of the n 3D points are distributed in x, y and z axes. A result of eigen decomposition of the covariance $\Sigma$ may be represented by $\Sigma A = A\Lambda$.

Column vectors of a matrix

$$A = \begin{bmatrix} w_{1p} w_{2p} w_{3p} \\ w_{1q} w_{2q} w_{3q} \\ w_{1r} w_{2r} w_{3r} \end{bmatrix}$$

consist of eigenvectors $w(p,q,r)$ of $\Sigma$. Diagonal elements of a diagonal matrix

$$\Lambda = \begin{bmatrix} \lambda_1 & 0 & 0 \\ 0 & \lambda_2 & 0 \\ 0 & 0 & \lambda_3 \end{bmatrix}$$

are eigenvalues $\lambda$ of $\Sigma$. Among $w = \{w_1, w_2, w_3\}$, the direction vector $w_3$ having the smallest eigenvalue $\lambda$ may be a direction from a tooth root to an occlusal surface (FIG. 4) or an opposite direction of the direction from the tooth root to the occlusal surface (FIG. 5). For example, in FIG. 3, $w_1$ having the largest eigenvalue $\lambda$ may be a direction connecting both outermost teeth in the lateral direction, $w_2$ having the second largest eigenvalue $\lambda$ may be a direction of a frontal direction of the patient or a rearward direction of the patient and $w_3$ having the smallest eigenvalue $\lambda$ may be a direction from the tooth root to the occlusal surface or the opposite direction. The direction vector $w_3$ may be expressed to $w_3=\{w_{3p},w_{3q},w_{3r}\}$.

An average of normal vectors $\overline{\eta}$ of a set of triangles of the 3D scan data may be used to find $w_3$ which is the direction from the tooth root to the occlusal surface. When $w_3 \cdot \overline{\eta} > 0$, $w_3$ may be determined as the projection direction. When $w_3 \cdot \overline{\eta} \leq 0$, $-w_3$ may be determined as the projection direction when generating the depth image. The projection plane is defined at a location separated by a predetermined distance from the 3D scan data with the projection direction vector as the normal vector.

In FIG. 4, the three axis directions of the 3D scan data obtained by the principal component analysis are $w_1$, $w_2$ and $w_3$, respectively. Among $w_1$, $w_2$ and $w_3$, the eigenvalue $\lambda$ of $w_1$ is the largest and the eigenvalue $\lambda$ of $w_3$ is the smallest. Herein, the projection direction may be determined using the direction vector $w_3$ having the smallest eigenvalue $\lambda$. When the teeth protrude upward, the average of the normal vectors of the set of the triangles of the 3D scan data represents an upward direction. In contrast, when the teeth protrude downward, the average of the normal vectors of the set of the triangles of the 3D scan data generated a downward direction. In FIG. 4, $w_3$ is substantially the same as the protruded direction of the teeth so that $w_3 \cdot \overline{\eta} > 0$ may be satisfied and $w_3$ may be used as the projection direction vector.

In FIG. 5, the three axis directions of the 3D scan data obtained by the principal component analysis are $w_1$, $w_2$ and $w_3$, respectively. Among $w_1$, $w_2$ and $w_3$, the eigenvalue $\lambda$ of $w_1$ is the largest and the eigenvalue $\lambda$ of $w_3$ is the smallest. Herein, the projection direction may be determined using the direction vector $w_3$ having the smallest eigenvalue $\lambda$. In FIG. 5, $w_3$ is substantially opposite to the protruded direction of the teeth so that $w_3 \cdot \overline{\eta} \leq 0$ may be satisfied and $-w_3$ may be used as the projection direction vector.

In this way, the projection direction is determined using the direction vector $w_3$ having the smallest eigenvalue $\lambda$ in the principal component analysis so that the 2D depth image may be well generated such that the teeth do not overlap with each other.

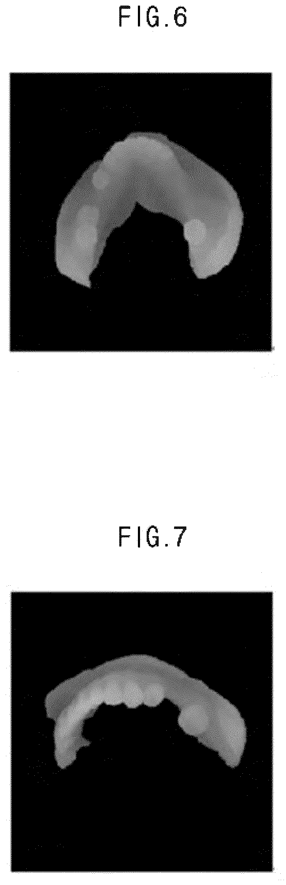

FIG. 6 is a plan view illustrating an example of the 2D depth image. FIG. 7 is a plan view illustrating an example of the 2D depth image.

FIGS. 6 and 7 are examples of the 2D depth image obtained by the operation of the generating the two-dimensional (2D) depth image (operation S100). A bright portion in the image indicates a portion having a great distance from the projection plane. A dark portion in the image indicates a portion having a little distance from the projection plane. The 2D depth image is an image having a depth value d for 2D coordinates {u, v}. The 3D scan data may be restored by back-projecting the 2D depth image in a direction opposite to the projection direction.

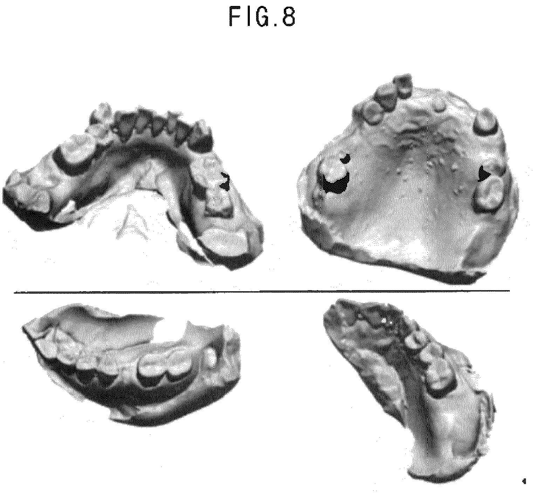

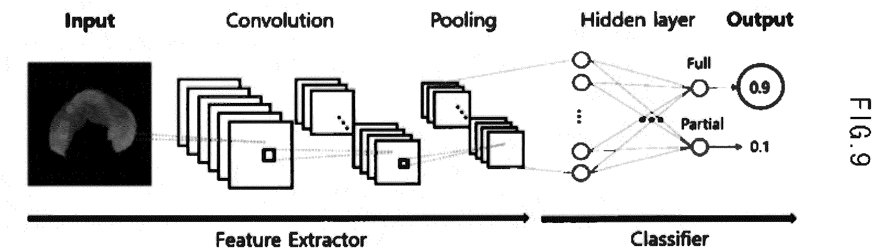

FIG. 8 is a perspective view illustrating full arch data and partial arch data. FIG. 9 is a conceptual diagram illustrating a convolutional neural network distinguishing the full arch data and the partial arch data.

Referring to FIGS. 1 to 9, the 3D scan data may be generated by varying a scan area according to a user's purpose. Data obtained by scanning all teeth of the patient may be referred to as the full arch data, and data obtained by scanning only a part of teeth of the patient may be referred to as the partial arch data. An upper part of FIG. 8 shows an example of the full arch data, and a lower part of FIG. 8 shows an example of the partial arch data.

A shape of the full arch data and a shape of the partial arch data are basically different from each other so that training steps for automatically detecting the landmarks of the full arch data and the partial arch data may be distinguished from each other and separate training models may be formed for the full arch data and the partial arch data. Thus, to completely automatically detect the landmark, a neural network model for classifying the full arch data and the partial arch data may be formed prior to the automatic landmark detection step.

A deep learning model may be generated using a convolutional neural network model receiving the 2D depth image generated in the operation of the generating the 2D depth image and arch classification information for classifying the full arch data and the partial arch data.

As shown in FIG. 9, the convolutional neural network model may include a feature extractor and a classifier. The input 2D depth image passes a feature extraction step including a convolution layer and a pooling layer so that the features are extracted from the input image. The convolution layer is a process of extracting features of the depth image, and the pooling layer is a process of culling the extracted features into several categories to classify them.

The classifier may calculate a score for arch classification information (full arch, partial arch) based on the feature extracted by the feature extractor. The input data is classified into an item having the highest score among items of the arch classification information.

As the extracted features pass through a hidden layer of the classifier, the scores for the items of the arch classification information are gradually extracted. As a result of passing all of the hidden layers, when a score for the full arch is higher than a score for the partial arch, the input depth image may be determined as the full arch data. In contrast, as a result of passing all of the hidden layers, when a score for the partial arch is higher than a score for the full arch, the input depth image may be determined as the partial arch data. In FIG. 9, the score for the full arch of the input depth image is 0.9 and the score for the partial arch of the input depth image is 0.1 so that the depth image may be determined as the full arch data.

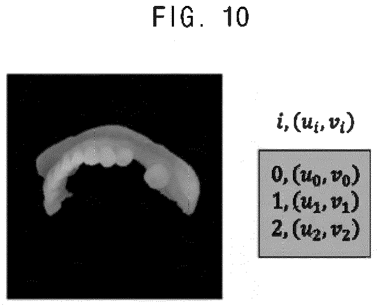

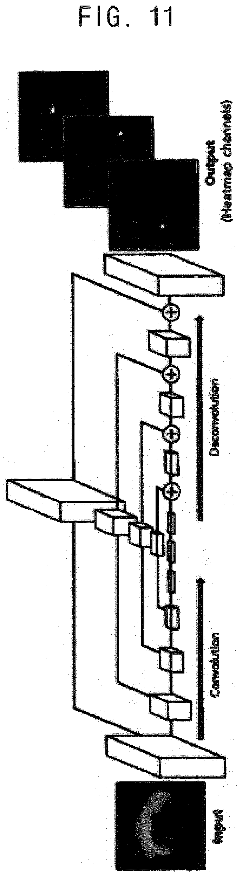

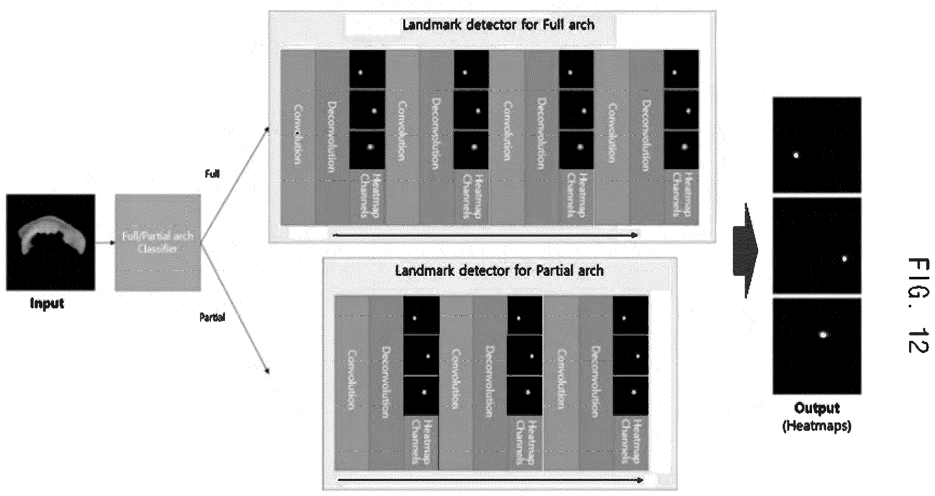

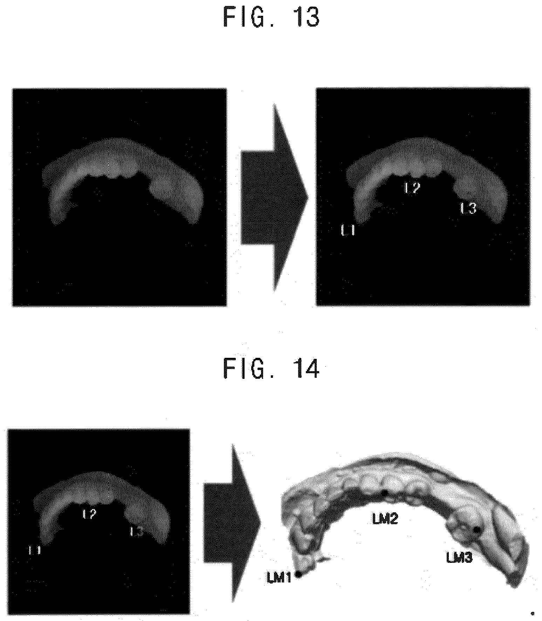

FIG. 10 is a conceptual diagram illustrating an example of training data of the convolutional neural network detecting a 2D landmark. FIG. 11 is a conceptual diagram illustrating the convolutional neural network detecting the 2D landmark. FIG. 12 is a conceptual diagram illustrating a first landmark detector for the full arch data and a second landmark detector for the partial arch data. FIG. 13 is a plan view illustrating an example of the 2D landmark.

Referring to FIGS. 1 to 13, a landmark deep learning model using a fully-connected convolutional neural network may be trained by receiving the depth image classified in the operation of determining the full arch data and the partial arch data (operation S200) and user-defined landmark information. As shown in FIG. 10, the user-defined landmark information used for the training may be 1) a type of the landmark to detect (e.g. index 0, 1 and 2) and 2) correct location coordinates (ui, vi) of the landmark in the 2D depth image.

The fully-connected convolutional neural network for the automatic landmark detection may be a neural network deep learning model including convolutional layers.

In the present embodiment, when the depth image is the full arch data, the automatic landmark detection may be operated using the fully-connected convolutional neural network trained using the full arch training data. In contrast, when the depth image is the partial arch data, the automatic landmark detection may be operated using the fully-connected convolutional neural network trained using the partial arch training data.

The fully-connected convolutional neural network may operate two major processes as shown in FIG. 11. In a convolution process, the feature of each landmark is detected and classified in the depth image through a plurality of pre-learned convolutional layers. By combining a result of the convolution process with entire image information in a deconvolution process, location information may be added to the feature and the location of the landmark on the image may be output as a heat map. The number of the output heat map images may be same as the number of user-defined landmarks defined when learning the deep learning model. For example, if the number of user-defined landmarks is three, three heat map images corresponding to the three landmarks may be output.

That is, the convolution process may be a process of extracting the features instead of losing location information from the 2D depth image. The feature of the landmark may be extracted through the convolution process. The deconvolution process may be a process of reviving lost location information for the landmark extracted in the convolution process.

In the present embodiment, the deep learning neural network model may include plural fully-connected convolutional neural networks which are iteratively disposed to enhance an accuracy of the detection.

The first landmark detector for the full arch data may include a first fully-connected convolutional neural network and the second landmark detector for the partial arch data may include a second fully-connected convolutional neural network.

The convolution process and the deconvolution process may be repeatedly operated in the first fully-connected convolution neural network model for the full arch data. The convolution process and the deconvolution process may be repeatedly operated in the second fully-connected convolution neural network model for the partial arch data. The number of the repeated operation of the convolution process and the deconvolution process in the first fully-connected convolution neural network model may be different from the number of the repeated operation of the convolution process and the deconvolution process in the second fully-connected convolution neural network model. For example, the number of the repeated operation of the convolution process and the deconvolution process in the first fully-connected convolution neural network model may be greater than the number of the repeated operation of the convolution process and the deconvolution process in the second fully-connected convolution neural network model.

As shown in FIG. 12, when the scan data is determined as the full arch data, four iterative neural networks (including four convolution processes and four deconvolution processes) may be generated

When the scan data is determined as the partial arch data, three iterative neural networks (including three convolution processes and three deconvolution processes) may be generated.

The depth image classified in the 3D scan data classification operation (operation S200) may be inputted to a system. The system may output the heat map indicating the location of the desired target landmark is output for each channel according to the user-defined landmark index of the learning model. A final result heat map may be obtained by adding all the output heat map data for the steps of the neural networks for each channel. The pixel coordinate having the largest value in the final result heat map data represents the location of the detected landmark. The heat map is output for each channel in the order of user-defined landmark index used during learning so that the location information of the desired landmark may be obtained.

FIG. 13 represents a result of automatically detecting landmarks of the 2D depth image using the fully-connected convolutional neural network model. The 2D landmarks in the 2D depth image are expressed as L1, L2 and L3.

FIG. 14 is a conceptual diagram illustrating a method of generating the 3D landmark by back-projecting the 2D landmark onto the 3D scan data.

Referring to FIGS. 1 to 14, the 2D coordinates of the landmarks L1, L2 and L3 obtained in the landmark automatic detection operation (operation S300) are converted into coordinates of landmarks LM1, LM2 and LM3 of the 3D scan data. The coordinates of the final 3D landmarks may be calculated using the projection information used in generating the depth image (operation S100). The 3D landmarks LM1 , LM2 and LM3 of the 3D scan data may be obtained by back-projecting the 2D landmarks L1, L2 and L3 onto the 3D scan data using the projection information used in generating the depth image (operation S100).

According to the present embodiment, the landmarks LM1, LM2 and LM3 in the 3D scan data are automatically detected using a deep learning so that effort and time for generating the landmarks LM1, LM2 and LM3 in the 3D scan data may be reduced and an accuracy of the landmark in the 3D scan data may be enhanced.

In addition, the landmarks LM1, LM2 and LM3 in the 3D scan data are automatically detected using a deep learning so that an accuracy of the registration of the dental CT image and the 3D scan data may be enhanced and time and effort for the registration of the dental CT image and the 3D scan data may be reduced.

According to an embodiment of the present inventive concept, a non-transitory computer-readable storage medium having stored thereon program instructions of the method for automatically detecting the landmark in 3D dental scan data may be provided. The above mentioned method may be written as a program executed on a computing device such as a computer. The method may be implemented in a general purpose digital computer which operates the program using a computer-readable medium. In addition, the structure of the data used in the above mentioned method may be written on a computer readable medium through various means. The computer readable medium may include program instructions, data files and data structures alone or in combination. The program instructions written on the medium may be specially designed and configured for the present inventive concept, or may be generally known to a person skilled in the computer software field. For example, the computer readable medium may include a magnetic medium such as a hard disk, a floppy disk and a magnetic tape, an optical recording medium such as CD-ROM and DVD, a magneto-optical medium such as floptic disc and a hardware device specially configured to store and execute the program instructions such as ROM, RAM and a flash memory. For example, the program instructions may include a machine language codes produced by a compiler and high-level language codes which may be executed by a computer using an interpreter or the like. The hardware device may be configured to operate as one or more software modules to perform the operations of the present inventive concept.

In addition, the above mentioned method for automatically detecting the landmark in 3D dental scan data may be implemented in a form of a computer-executed computer program or an application which are stored in a storage method.

[INDUSTRIAL AVAILABILITY]

The present inventive concept is related to the method for automatically detecting the landmark in 3D dental scan data and the non-transitory computer-readable storage medium having stored thereon program instructions of the method for automatically detecting the landmark in 3D dental scan data, effort and time for generating the landmarks in the 3D scan data may be reduced and effort and time for registration of the dental CT image and the digital impression model may be reduced.

Although a few embodiments of the present inventive concept have been described, those skilled in the art will readily appreciate that many modifications are possible in the embodiments without materially departing from the novel teachings and advantages of the present inventive concept. Accordingly, all such modifications are intended to be included within the scope of the present inventive concept as defined in the claims.

**Claims**

1. A method for automatically detecting a landmark in three-dimensional (3D) dental scan data, the method comprising:

   projecting 3D scan data to generate a two-dimensional (2D) depth image;
   determining full arch data obtained by scanning all teeth of a patient and partial arch data obtained by scanning only a part of teeth of the patient by applying the 2D depth image to a convolutional neural network model;
   detecting a 2D landmark in the 2D depth image using a fully-connected convolutional neural network model; and
   back-projecting the 2D landmark onto the 3D scan data to detect a 3D landmark of the 3D scan data.

2. The method of claim 1, wherein the projecting the 3D scan data comprises determining a projection direction vector by a principal component analysis.

3. The method of claim 2, wherein the determining the projection direction vector comprises:

   moving($X'$ = $X$ - $\overline{X}$) a matrix
   $$X = \begin{bmatrix} x_1 x_2 \cdots x_n \\ y_1 y_2 \cdots y_n \\ z_1 z_2 \cdots z_n \end{bmatrix}$$
   of a set $\{i \in \{1,2, \cdots , n\} \mid p_i(x_i, y_i, z_i)\}$ of coordinates of n 3D points of the 3D scan data based on an average value $\overline{X}$ of

$$X = \begin{bmatrix} x_1 & x_2 & \cdots & x_n \\ y_1 & y_2 & \cdots & y_n \\ z_1 & z_2 & \cdots & z_n \end{bmatrix};$$

calculating a covariance $\Sigma = cov(X') = \frac{1}{n-1}X'X'^T$ for the coordinates of the n 3D points;

operating ($\Sigma A = A\Lambda$) eigen decomposition of $\Sigma$; and

determining the projection direction vector based on a direction vector $w_3$ having the smallest eigenvalue $\lambda$ among $w_1=\{w_{1p},w_{1q},w_{1r}\}, w_2=\{w_{2p},w_{2q},w_{2r}\}, w_3=\{w_{3p},w_{3q},w_{3r}\}$,

where $A = \begin{bmatrix} w_{1p} & w_{2p} & w_{3p} \\ w_{1q} & w_{2q} & w_{3q} \\ w_{1r} & w_{2r} & w_{3r} \end{bmatrix}$ and

$\Lambda = \begin{bmatrix} \lambda_1 & 0 & 0 \\ 0 & \lambda_2 & 0 \\ 0 & 0 & \lambda_3 \end{bmatrix}$.

4. The method of claim 3, wherein the determining the projection direction vector comprises:

determining $w_3$ as the projection direction vector when $\overline{\eta}$ is an average of normal vectors of the 3D scan data and $w_3 \cdot \overline{\eta} > 0$; and

determining $-w_3$ as the projection direction vector when $\overline{\eta}$ is the average of the normal vectors of the 3D scan data and $w_3 \cdot \overline{\eta} \le 0$.

5. The method of claim 2, wherein the 2D depth image is generated on a projection plane, and the projection plane is defined at a location separated by a predetermined distance from the 3D scan data with the projection direction vector as a normal vector.

6. The method of claim 2, wherein the 2D landmark is back-projected in a direction opposite to the projection direction vector onto the 3D scan data to detect the 3D landmark.

7. The method of claim 1, wherein the convolutional neural network model comprises:

a feature extractor configured to extract a feature of the 2D depth image; and
a classifier configured to calculate a score for arch classification information based on the feature extracted by the feature extractor.

8. The method of claim 7, wherein the feature extractor comprises:

a convolution layer including a process of extracting features of the 2D depth image; and
a pooling layer including a process of culling the extracted features into categories.

9. The method of claim 1, wherein the detecting the 2D landmark comprises:

detecting the 2D landmark using a first fully-connected convolutional neural network model trained using full arch training data when the 2D depth image is the full arch data; and
detecting the 2D landmark using a second fully-connected convolutional neural network model trained using partial arch training data when the 2D depth image is the partial arch data.

10. The method of claim 9, wherein each of the first fully-connected convolutional neural network model and the second fully-connected convolutional neural network model operates:

a convolution process extracting a landmark feature from the 2D depth image; and
a deconvolution process adding landmark location information to the landmark feature.

11. The method of claim 10, wherein the convolution process and the deconvolution process are repeatedly operated in the first fully-connected convolution neural network model,

wherein the convolution process and the deconvolution process are repeatedly operated in the second fully-connected convolution neural network model, and
wherein a number of the repeated operation of the convolution process and the deconvolution process in the first fully-connected convolution neural network model is different from a number of the repeated operation of the convolution process and the deconvolution process in the second fully-connected convolution neural network model.

12. The method of claim 11, wherein the number of the repeated operation of the convolution process and the deconvolution process in the first fully-connected convolution neural network model is greater than the number of the repeated operation of the convolution process and the deconvolution process in the second fully-connected convolution neural network model.

13. The method of claim 1, wherein the detecting the 2D landmark further comprises training the convolutional neural network,

wherein the training the convolutional neural network comprises receiving a training 2D depth image and user-defined landmark information, and

wherein the user-defined landmark information includes a type of a training landmark and correct location coordinates of the training landmark in the training 2D depth image.

14. The method of claim 1, wherein the fully-connected convolutional neural network model operates:

a convolution process extracting a landmark feature from the 2D depth image; and

a deconvolution process adding landmark location information to the landmark feature.

15. The method of claim 14, wherein a result of the deconvolution process is a heat map corresponding to the number of the 2D landmarks.

16. The method of claim 15, wherein pixel coordinate having a largest value in the heat map represents a location of the 2D landmark.

17. A non-transitory computer-readable storage medium having stored thereon at least one program comprising commands, which when executed by at least one hardware processor, perform the method of claim 1.

# FIG. 1

START

PROJECTING 3D SCAN DATA TO GENERATE
2D DEPTH IMAGE — S100

DETERMINING FULL ARCH DATA AND PARTIAL ARCH
DATA BY APPLYING 2D DEPTH IMAGE TO
CONVOLUTIONAL NEURAL NETWORK — S200

DETECTING 2D LANDMARK BY APPLYING 2D DEPTH
IMAGE TO FULLY-CONNECTED CONVOLUTIONAL
NEURAL NETWORK — S300

BACK-PROJECTING 2D LANDMARK ONTO 3D SCAN DATA
TO DETECT 3D LANDMARK OF 3D SCAN DATA — S400

END

FIG. 2

FIG. 3

FIG. 4

FIG.5

FIG.6

FIG.7

FIG.8

FIG.9

FIG. 10

$i, (u_i, v_i)$

$0, (u_0, v_0)$
$1, (u_1, v_1)$
$2, (u_2, v_2)$

FIG. 11

FIG. 12

FIG. 13

FIG. 14

## INTERNATIONAL SEARCH REPORT

| International application No. |
| --- |
| **PCT/KR2020/018432** |

| A. | CLASSIFICATION OF SUBJECT MATTER |
| --- | --- |

**A61C 9/00**(2006.01)i; **G16H 30/40**(2018.01)i; **G16H 30/00**(2018.01)i; **A61B 5/00**(2006.01)i; **G06T 7/00**(2006.01)i; **G06N 3/08**(2006.01)i; **A61B 18/20**(2006.01)i

According to International Patent Classification (IPC) or to both national classification and IPC

| B. | FIELDS SEARCHED |
| --- | --- |

Minimum documentation searched (classification system followed by classification symbols)

A61C 9/00(2006.01); A61B 5/00(2006.01); A61B 6/00(2006.01); A61C 7/00(2006.01); G06K 9/00(2006.01); G06T 7/00(2006.01); G06T 7/11(2017.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 3차원(3-dimensional), 스캔(scan), 랜드마크(landmark), 2차원(2-dimensional), 깊이(depth), 완전 컨볼루션 신경망 모델(fully-connected convolutional neural network model), 풀 아치(full arch), 역투영(back projection)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| A | KR 10-2020-0023703 A (DDH INC.) 06 March 2020 (2020-03-06)<br>See paragraphs [0032]-[0094]; and figures 2-11. | 1-17 |
| A | KR 10-2019-0137388 A (OSSTEMIMPLANT CO., LTD.) 11 December 2019 (2019-12-11)<br>See paragraphs [0030]-[0072]; and figures 2-10. | 1-17 |
| A | KR 10-2014-0114308 A (SAMSUNG ELECTRONICS CO., LTD.) 26 September 2014 (2014-09-26)<br>See paragraphs [0040]-[0070]; and figures 1a-7. | 1-17 |
| A | US 2007-0081712 A1 (HUANG, Xiaolei et al.) 12 April 2007 (2007-04-12)<br>See paragraphs [0065]-[0150]; and figures 1-19. | 1-17 |
| A | KR 10-2020-0006506 A (DIO CORPORATION et al.) 20 January 2020 (2020-01-20)<br>See paragraphs [0040]-[0081]; and figures 1-8. | 1-17 |

☐ Further documents are listed in the continuation of Box C.     ☑ See patent family annex.

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **31 August 2021** | **31 August 2021** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**
Information on patent family members

International application No.

**PCT/KR2020/018432**

| Patent document cited in search report | | | Publication date (day/month/year) | Patent family member(s) | | | Publication date (day/month/year) |
|---|---|---|---|---|---|---|---|
| KR | 10-2020-0023703 | A | 06 March 2020 | CN | 111083922 | A | 28 April 2020 |
| | | | | EP | 3842005 | A1 | 30 June 2021 |
| | | | | JP | 2020-534037 | A | 26 November 2020 |
| | | | | KR | 10-2099390 | B1 | 09 April 2020 |
| | | | | US | 10991094 | B2 | 27 April 2021 |
| | | | | US | 2020-0286223 | A1 | 10 September 2020 |
| | | | | WO | 2020-040349 | A1 | 27 February 2020 |
| KR | 10-2019-0137388 | A | 11 December 2019 | KR | 10-2070256 | B1 | 29 January 2020 |
| KR | 10-2014-0114308 | A | 26 September 2014 | EP | 2977921 | A1 | 27 January 2016 |
| | | | | EP | 2977921 | A4 | 23 November 2016 |
| | | | | EP | 2977921 | B1 | 27 February 2019 |
| | | | | RU | 2013111932 | A | 27 September 2014 |
| | | | | US | 2016-0284089 | A1 | 29 September 2016 |
| | | | | US | 9799115 | B2 | 24 October 2017 |
| | | | | WO | 2014-148806 | A1 | 25 September 2014 |
| US | 2007-0081712 | A1 | 12 April 2007 | US | 7876938 | B2 | 25 January 2011 |
| | | | | WO | 2007-044508 | A2 | 19 April 2007 |
| | | | | WO | 2007-044508 | A3 | 02 August 2007 |
| KR | 10-2020-0006506 | A | 20 January 2020 | KR | 10-2277023 | B1 | 14 July 2021 |

Form PCT/ISA/210 (patent family annex) (July 2019)